# EUROPEAN PATENT APPLICATION

(11) **EP 1 380 265 A1**
(43) Date of publication of application: **14.01.2004**
(21) Application number: 03014567.6
(22) Date of filing: 07.07.2003
(51) Int. Cl.: A61B 17/22

(54) **Calculus treatment apparatus**

(30) Priority: 11.07.2002 JP 2002202738; 23.08.2002 JP 2002243921; 21.10.2002 JP 2002306097
(71) Applicant: Olympus Optical Corporation Limited, Tokyo 151-0072 (JP)
(72) Inventor: Nakamura, Takeaki, Hino-shi, Tokyo (JP); Hatori, Tsuruo, Sagamihara-shi, Kanagawa (JP); Sakurai, Tomohisa, Sagamihara-shi, Kanagawa (JP); Shimomura, Koji, Hachioji-shi, Tokyo (JP); Ono, Hiroo, Hino-shi, Tokyo (JP)
(74) Representative: von Hellfeld, Axel, Dr. Dipl.-Phys.,

(57) **Abstract**

A calculus treatment apparatus includes first (2) and second probe (3) which transmit first and second mechanical energy to a distal end side thereof and pulverize a calculus (A) by the first and second mechanical energy, and first and second mechanical energy generating devices which are arranged on a proximal end side of the first and second probes and generate the first and second mechanical energy. A probe arrangement structure is provided in which the first probe (2) and the second probe (3) are arranged substantially coaxially or concentrically.

## Description

### BACKGROUND OF THE INVENTION

### 1. Field of the Invention

The present invention relates to a calculus treatment apparatus for lithotripsy of a calculus formed in the coelom.

### 2. Description of the Related Art

Well-known conventional treatment means for lithotripsy of a calculus formed in the coelom includes one in which the lithotripsy is performed by ultrasonic vibration generated by an ultrasonic probe and one in which the lithotripsy is performed by a discharge operation. In the lithotripsy using the ultrasonic vibration, a probe main body includes an ultrasonic transducer by which the ultrasonic vibration is generated, then, is transmitted to the calculus by vibration transmitting member, and is subjected to the lithotripsy. In the lithotripsy using the discharge operation, a pair of electrodes are arranged at the distal end of a discharging probe and the discharge operation is executed between the electrodes for the lithotripsy.

However, both of the conventional means for the lithotripsy has drawbacks and advantages. Generally, the best lithotripsy apparatus is selected depending on the hardness and size of the calculus and the progress of the lithotripsy treatment. The above-mentioned different types of lithotripsy apparatuses are provided and alternatively used. Thus, the normal lithotripsy operation becomes complicated.

Next, Japanese Examined Patent Application Publication No. 57-8617 discloses a lithotripsy apparatus as a single apparatus which is commonly used for the lithotripsy using the discharge operation and the ultrasonic lithotripsy.

However, in the lithotripsy apparatus disclosed in Japanese Examined Patent Application Publication No. 57-8617, the discharging lithotripsy probe pulverizes the calculus by the discharge operation and thereafter another ultrasonic lithotripsy probe further pulverizes the calculus by ultrasonic waves. Consequently, the discharging lithotripsy probe needs to be pulled out from a sheath after the lithotripsy using the discharge operation and then the ultrasonic lithotripsy probe needs to be inserted in the sheath.

As mentioned above, the lithotripsy apparatus for both the discharging lithotripsy and the ultrasonic lithotripsy requires the lithotripsy means to be switched during the operation. Further, frequent exchange of the lithotripsy means becomes complicated and results in the increase in curative time for the lithotripsy.

A general lithotripsy apparatus transmits to the calculus, only single ultrasonic vibration generated by a transducer via a vibration transmitting stick. Therefore, in the case of a relatively hard calculus, the lithotripsy capacity produced by the ultrasonic vibration is not sufficient and calculus pulverization is not ensured.

Further, a conventional lithotripsy apparatus pulverizes the calculus by using two types of ultrasonic probes. However, in the conventional one, the same type of ultrasonic probes pulverizes the calculus and therefore a large calculus is not pulverized.

In addition, a mechanical shock lithotripsy apparatus is well known as one for lithotripsy using mechanical shock to the calculus. The mechanical shock lithotripsy apparatus has a high capacity for pulverizing, particularly, a large calculus. However, it has no lithotripsy capacity by which the calculus is sucked from the body.

Further, Japanese Unexamined Patent Application Publication No. 62-79049 discloses a lithotripsy apparatus having an ultrasonic probe and a discharging lithotripsy probe which are arranged adjacently to each other.

In the lithotripsy apparatus disclosed in Japanese Unexamined Patent Application Publication No. 62-79049, the ultrasonic probe and the discharging lithotripsy probe are formed adjacently to each other in the longitudinal direction.

Consequently, the probe cannot be made thin in diameter and a used portion thereof is limited. Further, the treatment positions at the distal end of the probe are varied and therefore the treatment becomes difficult.

Furthermore, U.S. Patent Publication No. 2002/0010478A1 discloses a lithotripsy apparatus in which an ultrasonic transducer alternately generates ultrasonic waves, and shock waves or compression waves.

However, the use of both the functions deteriorates in function as compared with that of a dedicated transducer. Thus, both of the functions are not simultaneously used.

### OBJECT OF THE INVENTION

It is an object of the present invention to provide a calculus treatment apparatus which has a thin diameter, pulverizes even a large calculus for a short time, and easily removes the calculus from the body.

### SUMMARY OF THE INVENTION

A calculus treatment apparatus includes: a first probe which transmits first mechanical energy to a distal end side thereof and pulverizes a calculus by the first mechanical energy; a first mechanical energy generating means which is arranged on a proximal end side of the first probe and generates the first mechanical energy; a second probe which transmits to a distal end side thereof, second mechanical energy different from the first mechanical energy and pulverizes the calculus by the second mechanical energy; and a second mechanical energy generating means which is arranged on a proximal end side of the second probe and generates the second mechanical energy different from the first mechanical energy, wherein a probe arrangement structure is provided in which the first probe and the second probe are arranged nearly coaxially or concentrically.

### BRIEF DESCRIPTION OF THE DRAWINGS

Fig. 1 is an explanatory diagram showing the entire structure including a calculus treatment apparatus according to a first embodiment of the present invention;
Fig. 2 is a circuit diagram of a driving system for driving the calculus treatment apparatus shown in Fig. 1;
Figs. 3A and 3B are explanatory diagrams showing a relationship between a distal end position of an ultrasonic lithotripsy probe and a distal end position of a mechanical lithotripsy probe in the calculus treatment apparatus according to the first embodiment;
Figs. 4A to 4G are explanatory diagrams showing various distal end shapes of the mechanical lithotripsy probe shown in Figs. 3A and 3B;
Fig. 5 is a longitudinal cross-sectional view showing a calculus treatment apparatus according to a second embodiment of the present invention;
Figs. 6A to 6C are explanatory diagrams showing a relationship between a distal end position of an ultrasonic lithotripsy probe and a distal end position of a mechanical lithotripsy probe in the calculus treatment apparatus according to the second embodiment;
Fig. 7 is a diagram showing the entire structure of a calculus treatment apparatus according to a third embodiment of the present invention;
Fig. 8 is a diagram showing the structure of a distal end side of an ultrasonic lithotripsy probe forming the calculus treatment apparatus according to the third embodiment;
Fig. 9 is a diagram showing a state in which the ultrasonic lithotripsy probe forms a hole in a calculus;
Fig. 10 is a diagram showing a state in which after forming a hole deeper than that shown in Fig. 9, the distal end side of a mechanical shock lithotripsy probe is inserted in the hole;
Fig. 11 is a diagram showing the entire structure of a calculus treatment system according to a fourth embodiment of the present invention;
Fig. 12 is a diagram showing the entire structure of a calculus treatment system according to a fifth embodiment of the present invention;
Fig. 13 is a cross-sectional view showing a hand piece portion forming the calculus treatment apparatus according to the fifth embodiment;
Fig. 14 is a cross-sectional view of an A-A line shown in Fig. 13;
Fig. 15 is a cross-sectional view showing a hand piece portion according to a sixth embodiment of the present invention;
Fig. 16 is a cross-sectional view showing a hand piece portion according to a seventh embodiment of the present invention;
Fig. 17 is a cross-sectional view showing a hand piece portion according to an eighth embodiment of the present invention; and
Fig. 18 is a cross-sectional view showing a hand piece portion according to a ninth embodiment of the present invention.

### DESCRIPTION OF THE PREFERRED EMBODIMENTS

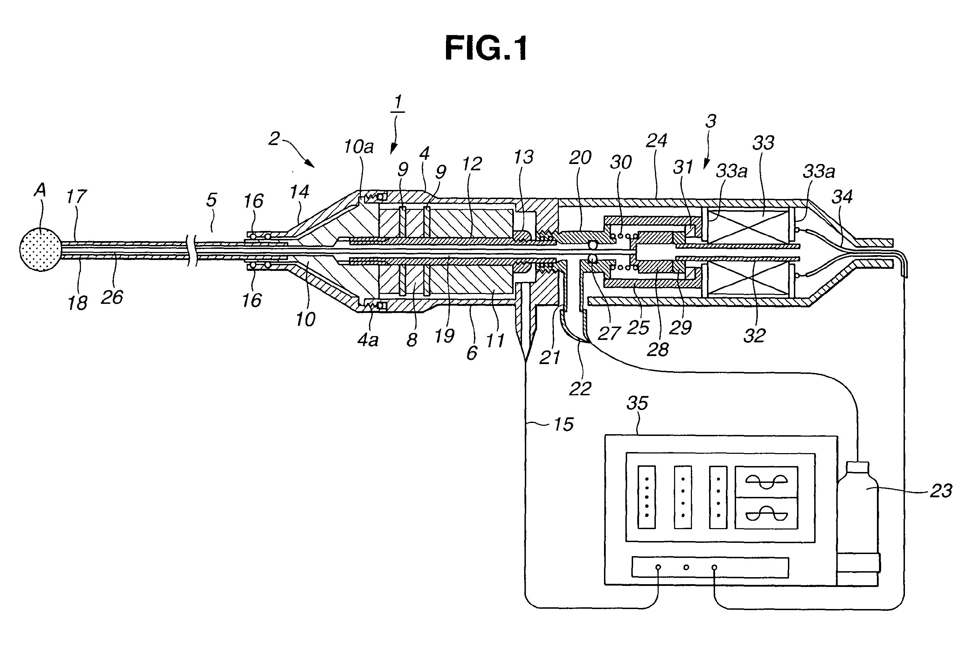

A calculus treatment apparatus will be described according to a first embodiment of the present invention with reference to Figs. 1 to 4G.

Fig. 1 shows the entire structure of a calculus treatment system having a calculus treatment apparatus 1 according to the first embodiment. The calculus treatment apparatus 1 is integrated as a single apparatus by combining an ultrasonic lithotripsy probe 2 for mechanical lithotripsy using ultrasonic vibration and a mechanical lithotripsy probe 3 for lithotripsy using mechanical shock caused by magnetic force.

The ultrasonic lithotripsy probe 2 includes a grip portion 4 which is gripped by an operator and a long inserting portion 5 which is inserted in the coelom. The inserting portion 5 is projected in a straight line from the grip portion 4. The grip portion 4 has a cylindrical case 6. The case 6 has a Langevin type transducer 7 which generates ultrasonic vibrating energy.

In the Langevin type transducer 7, a piezoelectric element 8 and an electrode 9 are overlapped, then they are sandwiched between a horn 10 serving as a front metal block and a rear metal block 11, and they are coupled to the horn 10. Further, a nut 13 is screwed to a back end of a bolt 12 which pierces through the rear metal block 11, thereby tightening the layered piezoelectric element 8. The electrode 9 is connected to a power supply cord 15 which leads to the outside.

The case 6 in the grip portion 4 is closely fit to the furthest outer peripheral portion of the horn 10 only at the front end thereof and coaxially supports the horn 10. A male portion 4a is formed to a front-end outer periphery of the case 6, and a proximal end of an exterior cap 14 for covering the horn 10 is twisted in the male screw portion 4a.

A flange 10a arranged at the furthest outer peripheral portion of the horn 10 is sandwiched between the horn 10 and the exterior cap 14, thereby positioning and fixing the horn 10. Thus, a positional relationship is determined between the horn 10 and the exterior cap 14. The exterior cap 14 covers the outer peripheral portion of the horn 10 in a noncontact state.

An elastic O-shaped ring 16 is arranged between the outer periphery of a distal end portion of the horn 10 and an inner surface of the exterior cap 14 and, consequently, the elastic O-shaped ring 16 closely seals a gap therebetween and the distal end portion of the horn 10 is elastically supported.

A vibration transmitting member 17 comprising a metal hollow pipe forming the inserting portion 5 is fixedly attached to the distal end of the horn 10. The horn 10 is coaxially coupled to the vibration transmitting member 17 and ultrasonic vibrations (mechanical energy) amplified by the horn 10 are transmitted to the vibration transmitting member 17.

An inner hole (hollow hole) 18 of the vibration transmitting member 17 is connected to a through-hole 19 which is formed to horizontally pierce through the center of the horn 10 and the bolt 12. The through-hole 19 forms a suction path for suction and evacuation of the calculus which is pulverized. A back end of the through-hole 19 is connected to a hollow coupling member 20 fixed to the back end of the case 6 in the grip portion 4 by screwing.

A suction cap 21 is arranged against a side wall of the coupling member 20. A suction tube 22 is connected to the suction cap 21 and the suction tube 22 is extended to a suction pump 23 shown in Fig. 1 and is connected to the suction pump 23.

The mechanical lithotripsy probe 3 is detachably coupled to the coupling member 20. The mechanical lithotripsy probe 3 is detachably fixed to the back end portion of the grip portion 4 in the ultrasonic lithotripsy probe 2 by screw-type or bayonet-type detaching and coupling means. Specifically, a coil fixing member 25 on the mechanical lithotripsy probe 3 is detachably attached by the screw coupling to the back end portion of the coupling member 20 in the ultrasonic lithotripsy probe 2.

The coupling member 20 in the ultrasonic lithotripsy probe 2 includes, at the back end portion thereof, a bearing member 27 comprising an O-shaped ring which supports the back end portion of a long lithotripsy probe 26 movably in the long-axis direction. The distal end portion of the lithotripsy probe 26 extends to and pierces through the vibration transmitting member 17, and is projected to the outside from the distal end of the vibration transmitting member 17.

The back end of the lithotripsy probe 26 is attached and fixed to an electrical insulating relay member 28 for preventing leakage current, which is arranged in the coil fixing member 25. The relay member 28 and the ring 29 form an integral block by fixing the metal ring 29 to the back end of the relay member 28. A coil spring 30 exists between the front end of the relay member 28 and the coupling member 20 in front thereof. The metal ring 29 is energized backward by the coil spring 30.

Referring to Fig. 1, the ring 29 integral with the lithotripsy probe 26 and the relay member 28 is energized in a returning direction by energization force of the coil spring 30. Normally, the ring 29 strikes a buffer 31 attached to the coil fixing member 25 and stops and waits at the striking position. A free length (height) of the coil string 30 is set so as to strike to the buffer 31.

On the other hand, a power supply cord 34 connected to the electro-magnetic coil 33 and the power supply cord 15 are led to an energization control device 35 shown in Fig. 1 and are connected to a driving circuit of the energization control device 35.

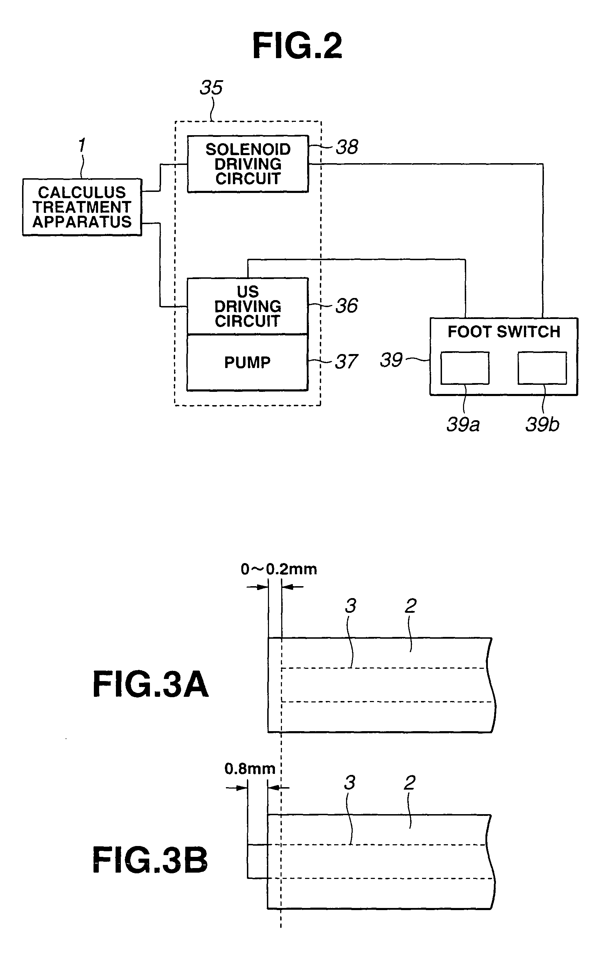

Fig. 2 is a block diagram showing circuits for controlling the driving of the calculus treatment apparatus 1 shown in Fig. 1. The energization control device 35 comprises a US driving circuit 36 which drives the ultrasonic lithotripsy probe 2, a pump driving circuit 37 which sucks the pulverized calculus so that it can be sucked by the ultrasonic lithotripsy probe 2 driven by the US driving circuit 36, and a solenoid driving circuit 38 which drives the mechanical lithotripsy probe 3.

A foot switch 39 serving as driving operation means is connected to the energization control device 35. The lithotripsy probe 2 or 3 is arbitrarily and selectively driven by selecting and operating any of a switch 39a for operating the US driving circuit 36 and the pump driving circuit 37 and a switch 39b for operating the solenoid driving circuit 38 in the foot switch 39.

Next, a description is given of a positional relationship between a distal end position of the ultrasonic lithotripsy probe 2 and a distal end position of the mechanical lithotripsy probe 3 with reference to Figs. 3A and 3B.

In order to improve the efficiency of lithotripsy and to reduce the lithotripsy time by using both functions of the ultrasonic lithotripsy probe 2 and the mechanical lithotripsy probe 3, generally, it is preferable that the distal ends of the ultrasonic lithotripsy probe 2 and the mechanical lithotripsy probe 3 are arranged so that the stroke width of ultrasonic vibration at the distal end of the ultrasonic lithotripsy probe 2 entirely covers a moving stroke width upon lithotripsy of the distal end of the mechanical lithotripsy probe 3 or it covers at least a part thereof.

A description is given of the positional relationship between the distal end position of the ultrasonic lithotripsy probe 2 and the distal end position of the mechanical lithotripsy probe 3, assuming that the amount of movement of vibrations L1 caused by the ultrasonic lithotripsy probe 2 is 0.1 mm or less and the amount of movement L2 caused by the mechanical lithotripsy probe 3 is 1.0 mm.

Referring to Fig. 3A, upon turning off both the ultrasonic lithotripsy probe 2 and the mechanical lithotripsy probe 3, a distal end surface of the mechanical lithotripsy probe 3 is on the same plane as that of a distal end surface of the ultrasonic lithotripsy probe 2 or is away from the distal end surface of the mechanical lithotripsy probe 3 by 0.2 mm in the hand direction. In such a state, driving power for coarse lithotripsy of a calculus A is supplied to the mechanical lithotripsy probe 3. Then, referring to Fig. 3B, the distal end surface of the mechanical lithotripsy probe 3 is projected from the distal end surface of the ultrasonic lithotripsy probe 2 by 1.0 to 0.8 mm and, consequently, the calculus A is mechanically pulverized.

Subsequently, the power is supplied to finely pulverize the coarsely pulverized calculus A which can be evacuated from the body. Then, the distal end surface of the ultrasonic lithotripsy probe 2 is moved from the position shown in Fig. 3A by 0.1 mm and the ultrasonic vibrations due to this movement finely pulverize the calculus A so that it can be evacuated. In this case, the distal end surface of the mechanical lithotripsy probe 3 is at the position which does not interfere to the lithotripsy operation caused by the ultrasonic vibration.

Preferably, upon simultaneously driving the ultrasonic lithotripsy probe 2 and the mechanical lithotripsy probe 3, the distal end surface of the mechanical lithotripsy probe 3 is at the position back to the distal end surface of the ultrasonic lithotripsy probe 2, as shown in Fig. 3A.

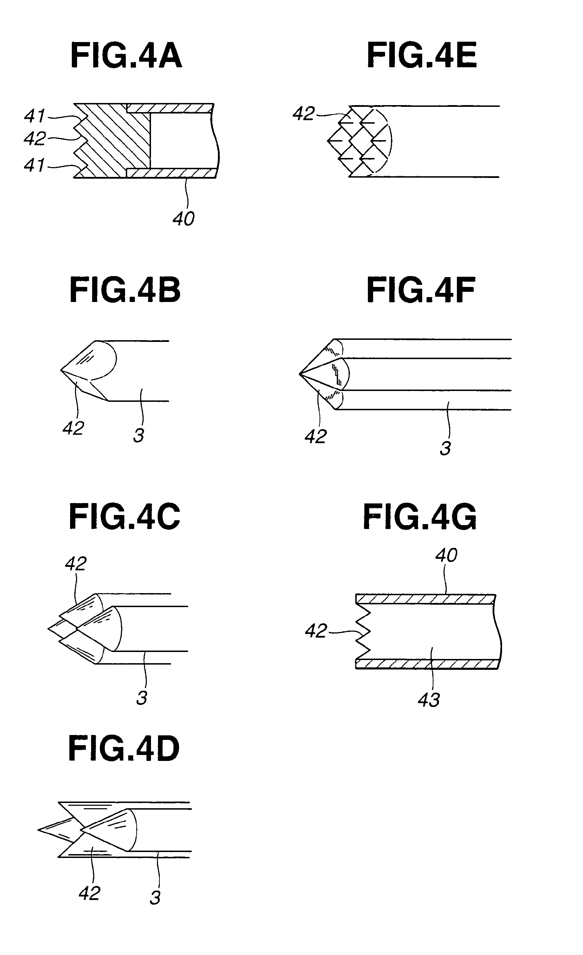

Next, a description is given of the shape types of the distal end portion of the mechanical lithotripsy probe 3 with reference to Figs. 4A to 4G.

Referring to Fig. 4A, the mechanical lithotripsy probe 3 comprises a pipe 40, a plurality of slits 41 are formed at a distal end surface of the pipe 40, and a strike portion 42 for striking the calculus A is formed at the distal end of the pipe 40. In particular, the distal end shape of the mechanical lithotripsy probe 3 may be projected as shown in Figs. 4B to 4F.

Fig. 4B shows a one-line knife-shaped distal end portion of the mechanical lithotripsy probe 3, Fig. 4C shows the distal end portion of the mechanical lithotripsy probe 3 having a plurality of sharp projections concentratedly arranged in the center, Fig. 4D shows the distal end portion of the mechanical lithotripsy probe 3 having a plurality of sharp projections arranged at the periphery, Fig. 4E shows the distal end portion of the mechanical lithotripsy probe 3 having a large number of small projections, and Fig. 4F shows the distal end portion of the mechanical lithotripsy probe 3 having a blade with a distal end.

Fig. 4G shows the distal end portion of the mechanical lithotripsy probe 3 comprising the pipe 40 whose distal end is opened to allow the flowing of a solution for cooling. At the distal end portion of the mechanical lithotripsy probe 3 having a plurality of projections shown in Fig. 4G, the calculus or drainage in the coelom may be sucked via the hollow hole 43. Alternatively, the calculus A may be pulverized by driving only the ultrasonic lithotripsy probe 2 depending on the state of the calculus A.

According to the first embodiment, the single calculus treatment apparatus is formed by combining two types of probes having different pulverizing capacities and, thus, the proper pulverization can effectively be performed without switching the two types of probes.

The pulverizing capacity of one probe is set so that the calculus is finely pulverized by suction through the sucking path of the other probe and the pulverized calculus can efficiently be sucked. In addition, particularly large calculus A can be pulverized for a short time and it can be evacuated from the body.

Magnetic force generated by the energization of the electro-magnetic coil 33 reciprocatedly vibrates an iron core 32 and mechanical energy transmitted to the lithotripsy probe 26 is generated according to the first embodiment. However, the iron core 32 is fixed and the electro-magnetic coil 33 is energized by using the ring 29 as a magneto (magnetic material), the generated magnetic force reciprocatedly vibrates the ring 29, and the mechanical energy may be transmitted to the lithotripsy probe 26.

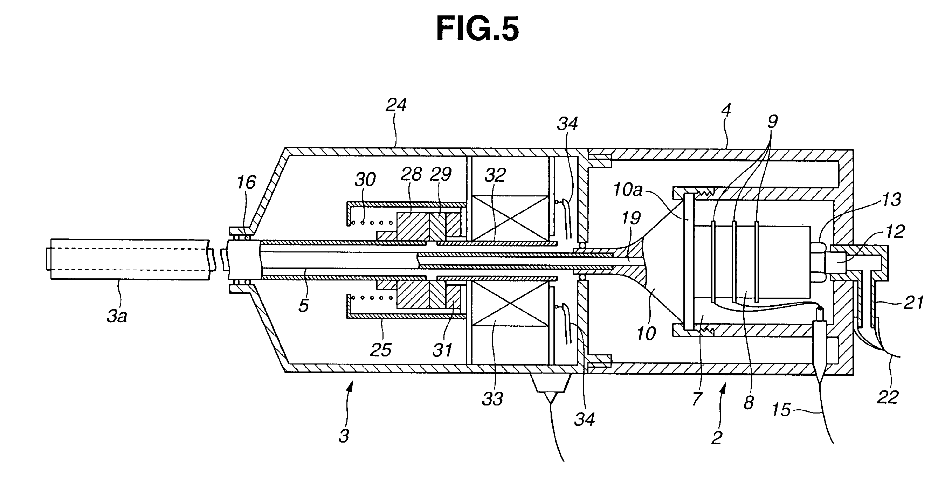

A description is given of a calculus treatment apparatus according to a second embodiment of the present invention with reference to Figs. 5 to 6C.

Unlike the first embodiment, the positions of the ultrasonic lithotripsy probe 2 and the mechanical lithotripsy probe 3 are reversed back and forth according to the second embodiment. A component having the same function as that of the first embodiment is designated by the same reference numeral and it is not described in detail.

The ultrasonic lithotripsy probe 2 has the pipe-shaped inserting portion 5, a luminal portion of the inserting portion 5 is set as a suction path for sucking the pulverized calculus, and the pulverized calculus is evacuated to the outside of the body via the suction cap 21.

The mechanical probe 3 has a pipe-shaped inserting portion 3a. The inserting portion 5 of the ultrasonic lithotripsy probe 2 is inserted into a hollow hole of the inserting portion 3a so that the mechanical lithotripsy probe 3 is coaxially arranged to the ultrasonic lithotripsy probe 2. A front end portion of the case 6 in the grip portion 4 in the ultrasonic lithotripsy probe 2 is coupled to the back end of the grip portion 24 in the mechanical probe 3 by screw-type or bayonet-type detaching and coupling means. Thus, the mechanical lithotripsy probe 3 is detachably coupled and fixed to the ultrasonic lithotripsy probe 2.

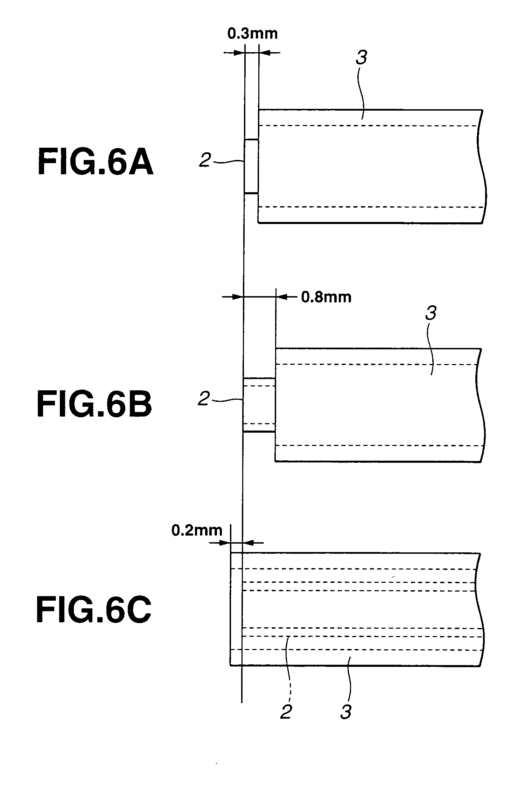

Figs. 6A to 6C show a relationship between the distal end positions of the ultrasonic lithotripsy probe 2 and the mechanical lithotripsy probe 3.

A description is given of the positional relationship between the ultrasonic lithotripsy probe 2 and the mechanical lithotripsy probe 3, assuming that the amount of movement of vibrations L1 caused by the ultrasonic lithotripsy probe 2 is 0.1 mm or less and the amount of movement L2 caused by the mechanical lithotripsy probe 3 is 1.0 mm.

Referring to Fig. 6A, upon turning off both the ultrasonic lithotripsy probe 2 and the mechanical lithotripsy probe 3, a distal end surface of the mechanical lithotripsy probe 3 is on the same plane as that of a distal end surface of the ultrasonic lithotripsy probe 2 or is away from the distal end surface of the mechanical lithotripsy probe 3 by 0.3 mm in the hand direction.

In such a state, driving power for coarse lithotripsy of the calculus A is supplied to the mechanical lithotripsy probe 3. Then, referring to Fig. 6B, the distal end surface of the mechanical lithotripsy probe 3 is projected from the distal end surface of the ultrasonic lithotripsy probe 2 by 1.0 mm to 0.8 mm and, consequently, the calculus A is mechanically pulverized.

Subsequently, the power is supplied to finely pulverize the coarsely pulverized calculus A which can be evacuated from the body. Then, the distal end surface of the ultrasonic lithotripsy probe 2 is moved from the position shown in Fig. 6A by 0.1 mm and the ultrasonic vibrations due to this movement finely pulverizes the calculus A so that it can be evacuated.

In this case, preferably, the distal end surface of the mechanical lithotripsy probe 3 is at the position which does not interfere to the lithotripsy operation caused by the ultrasonic vibration. Upon simultaneously driving the ultrasonic lithotripsy probe 2 and the mechanical lithotripsy probe 3, the distal end surface of the mechanical lithotripsy probe 3 is at the position in front of the distal end surface of the ultrasonic lithotripsy probe 2, as shown in Fig. 6C. Alternatively, the distal end surface of the mechanical lithotripsy probe 3 may be on the same plane as that of the distal end surface of the ultrasonic lithotripsy probe 2. The calculus A may be positioned so that it comes into contact with the distal ends of both the mechanical lithotripsy probe 3 and the ultrasonic lithotripsy probe 2 and the calculus A may be pulverized thereby.

The calculus treatment apparatus 1 according to the first and second embodiments is formed by combining the ultrasonic lithotripsy probe 2 and the mechanical lithotripsy probe 3 and coaxially arranging them back and forth. However, the coaxial arrangement does not mean a strictly mathematical relationship.

The present invention is not limited to the above first and second embodiments and can be applied to another embodiment. For example, as means for generating the mechanical energy of the mechanical lithotripsy probe 3, a vibrating plate which is vibrated by the magnetic force of the solenoid may be used so that the probe may be vibrated.

As mentioned above, in the calculus treatment apparatus according to the first and second embodiments, the proper lithotripsy can efficiently be executed for a short time in accordance with the state of the calculus without exchanging the probe because of coaxial arrangement of two or more types of probes having the different lithotripsy capacities. Advantageously, the load of an operator and a patient is reduced.

Further, the probes have the thinner diameter by coaxially arranging the two or more types of probes having the different lithotripsy capacities and can widely be used. The treatment positions of the probe distal ends are substantially the same and, upon lithotripsy, the distal end position of the ultrasonic lithotripsy probe 2 does not need to be deviated from the distal end position of the mechanical lithotripsy probe 3. Thus, the lithotripsy becomes easy.

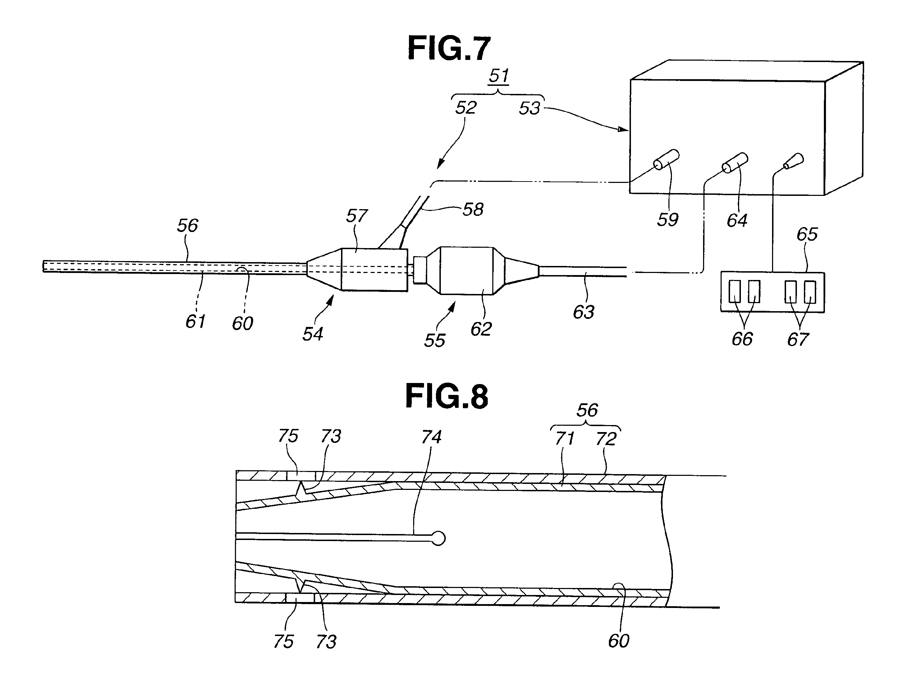

Next, a description is given of a third embodiment of the present invention with reference to Figs. 7 to 10.

Referring to Fig. 7, a calculus treatment system 51 comprises a calculus treatment apparatus 52 and a probe driving device 53 connected to the calculus treatment apparatus 52, for applying a driving signal thereto according to the third embodiment.

The calculus treatment apparatus 52 comprises an ultrasonic lithotripsy probe 54 and a mechanical shock lithotripsy probe 55 which can be attached to the ultrasonic lithotripsy probe 54.

The ultrasonic lithotripsy probe 54 comprises an elongated, cylindrical, and hollow probe portion 56 and a transducer portion 57 which is arranged at the back end in the hand side of the probe portion 56 and which incorporates an ultrasonic transducer (transducer). A signal cable 58 connected to the ultrasonic transducer is extended from the side portion of the transducer portion 57, and a connector 59 arranged at the end portion of the signal cable 58 is detachably connected to a first connector supporter arranged to the probe driving device 53.

The ultrasonic lithotripsy probe 54 has an inserting hole 60 in which a probe portion 61 of the mechanical shock lithotripsy probe 55 can be inserted along the longitudinal direction. The back end of the inserting hole 60 is opened at the back end of the transducer portion 57. The probe portion 61 of the mechanical shock lithotripsy probe 55 is inserted or led out from the opening so as to be detachable.

The opening portion has, e.g., a side groove. A projection portion arranged to the probe portion 61 is engaged in the side groove and is attached without falling by inserting and rotating the probe portion 61. A longitudinal groove is arranged in a deep portion of the side groove and the projection portion can move in the longitudinal direction of the probe portion 61 with a predetermined stroke.

As mentioned above, the probe portion 56 in the ultrasonic lithotripsy probe 54 is formed with the hollow structure and the probe portion 61 is coaxially arranged to the probe portion 56 by inserting the probe portion 61 of the mechanical shock lithotripsy probe 55.

On the other hand, the mechanical shock lithotripsy probe 55 comprises the probe portion 61 which is made of, e.g., a metal with an elongated and solid structure and which can be inserted in the inserting hole 60 of the ultrasonic lithotripsy probe 54, and a solenoid portion 62 which is arranged on the back end in the hand side portion of the probe portion 61 and which incorporates a solenoid. A signal cable 63 connected to the solenoid is extended from, e.g., the back end of the solenoid portion 62, and a second connector 64 arranged at the end portion of the signal cable 63 is detachably connected to a second connector supporter arranged to the probe driving device 53.

A foot switch 65 is connected to the probe driving device 53. The lithotripsy using the ultrasonic vibration caused by the ultrasonic lithotripsy probe 54 and the lithotripsy using the shock waves caused by the mechanical shock lithotripsy probe 55 are performed by pressing a switch 66 for the ultrasonic lithotripsy probe which is arranged to the foot switch 65 and a switch 67 for the mechanical shock lithotripsy probe.

Two switches 66 for the ultrasonic lithotripsy probe 54 and two switches 67 for the mechanical shock lithotripsy probe 55 are provided. One switch of the two switches 66 and one switch of the two switches 67 are ON switches for outputting the driving signal. Another switch of the two switches 66 and another switch of the two switches 67 are OFF switches for stopping the output of the driving signal.

In this case, both the ultrasonic lithotripsy probe 54 and the mechanical shock lithotripsy probe 55 can be combined in the longitudinal direction while the ultrasonic lithotripsy probe 54 is sheath-shaped and the mechanical shock lithotripsy probe 55 is knife-shaped.

According to the third embodiment, it is possible to detachably combine and use the two probes which generate different mechanical energy.

Fig. 8 shows a cross-sectional view showing the probe portion 56 in the ultrasonic lithotripsy probe 54. The probe portion 56 comprises an inner cylindrical tube 71 on the inside and an outer cylindrical tube 72 on the outside. The distal end side of the inner cylindrical tube 71 is narrower toward the distal end (that is, having a smaller diameter as it is near the distal end) and a plurality of spike-shaped projections 73 are projected to the outside at the position slightly far away from the distal end.

On the distal end side of the inner cylindrical tube 71, a plurality of notches (grooves) 74 cut in the longitudinal direction are arranged from the distal end to the hand portion. The proximal ends of the notches 74 substantially match to the starting position of the narrow diameter.

On the other hand, an opening 75 is arranged at a position corresponding to the projection 73 in the outer cylindrical tube 72.

The ultrasonic vibrations generated by the transducer portion 57 are transmitted to the probe portion 56. The outer diameter of the probe portion 61 in the mechanical shock lithotripsy probe 55 is slightly smaller than the inner diameter of the inner cylindrical tube 71 and can be inserted in the inner cylindrical tube 71. The solenoid driving portion 62 promptly emits the probe portion 61 in the distal end direction (or, the solenoid driving portion 62 drives the probe portion 61 so that it is shockingly projected in the distal end direction with an amplitude larger than that of the ultrasonic vibration).

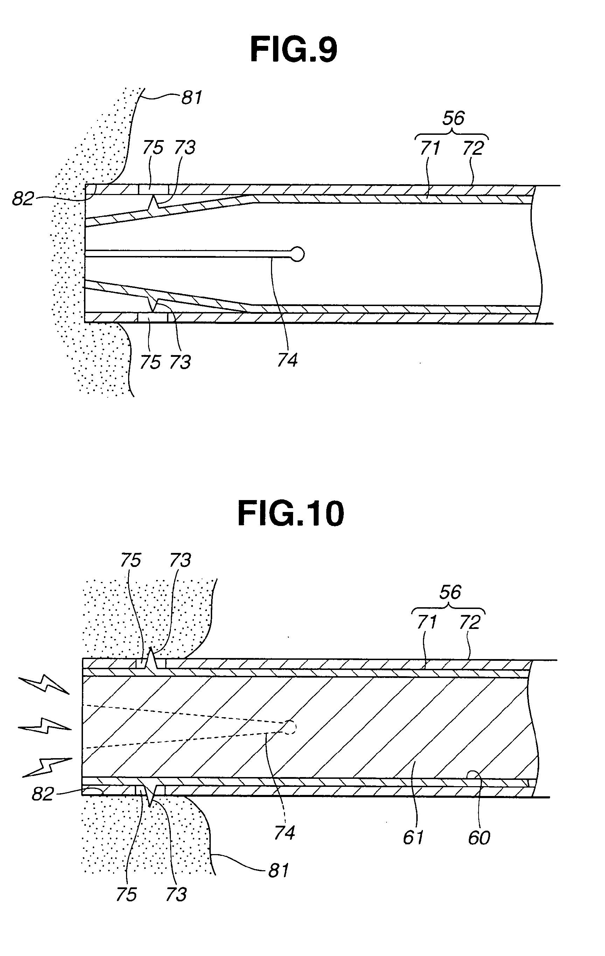

The probe portion 61 is used by being inserted in the hollow probe portion 56 (Fig. 10 shows a state in which the probe portion 61 of the mechanical shock lithotripsy probe 55 is inserted in the probe portion 56).

In the state of combining the ultrasonic lithotripsy probe 54 and the mechanical shock lithotripsy probe 55, the distal end position of the probe portion 56 is the same as that of the probe portion 61 or the distal end portion of the probe portion 61 protrudes slightly.

Next, the operation will be described according to the third embodiment.

First, the distal end of the probe portion 56 in the ultrasonic lithotripsy probe 52 is stroke to the calculus and the ON switch of the switch 66 for the ultrasonic lithotripsy in the foot switch 65 is operated. Thus, the driving signal for energizing the ultrasonic vibration is applied to the ultrasonic transducer in the transducer portion 62 from the probe driving device 53 and the ultrasonic transducer is ultrasonically vibrated.

The probe portion 56 transmits the ultrasonic vibration and the distal end of the probe portion 56 deeply enters the calculus so that it pierces through the calculus. Fig. 9 shows a state in which a small hole 82 is formed to a calculus 81.

When the distal end of the probe portion 56 enters the calculus 81 at some degree, for example, in a state shown in Fig. 10, the probe portion 61 of the mechanical shock lithotripsy probe 55 is inserted. The distal end portion of the inner cylindrical tube 71 is widened by the distal end of the probe portion 61 in accordance with the insertion of the distal end of the probe portion 61 to the proximal end portion having the narrow diameter. Further, the distal end of the inner cylindrical tube 71 is inserted in the opening 75 in accordance with the modification thereof, the projection 73 is projected in the side direction, and the projection 73 is stopped to the calculus 81 (in other words, the distal end side of the calculus treatment apparatus 52 is stopped to the calculus 81 by the projection 73).

After that, the solenoid driving portion 62 is driven and mechanical shock waves are generated. The shock is transmitted to the probe portion 61 and is effectively transmitted to the calculus 81 to which the distal end of the probe portion 61 is stopped. That is, since the projection 73 stops the calculus 81, the shock is not released and is certainly transmitted to the calculus 81. Then, the calculus 81 is effectively pulverized.

As mentioned above, when the large calculus 81 is pulverized into small calculuses, the switch 66 for the ultrasonic lithotripsy probe may further be operated. Thus, the calculuses are pulverized by the ultrasonic waves to have the size to be removed to the outside of the body.

The lithotripsy using the ultrasonic vibration is not necessary for the calculus having the size smaller than that to be removed to the outside of the body by the mechanical shocks.

Although the large calculus is pulverized, the small calculus can be pulverized by only the ultrasonic lithotripsy probe 54.

In other words, according to the third embodiment, the small calculus is pulverized. In addition, the large calculus is pulverized by the mechanical shock lithotripsy probe 55 inserted in the ultrasonic lithotripsy probe 54. After that, the calculus is pulverized to have the size to be removed by the ultrasonic lithotripsy probe 54 if necessity.

Since the positional relationship between the calculus and the distal end of the probe for the mechanical shock lithotripsy is maintained according to the third embodiment, the large calculus is certainly pulverized. The calculus is divided into block pieces and then the pieces are pulverized by the ultrasonic waves while preventing losing sight of the target without pulling in and out the probe. Thus, it is possible to provide the calculus treatment apparatus for efficiently pulverizing the large calculus.

By using the lithotripsy, the operation time for the lithotripsy is reduced and the physical and psychological burden of the patient and the operator is reduced.

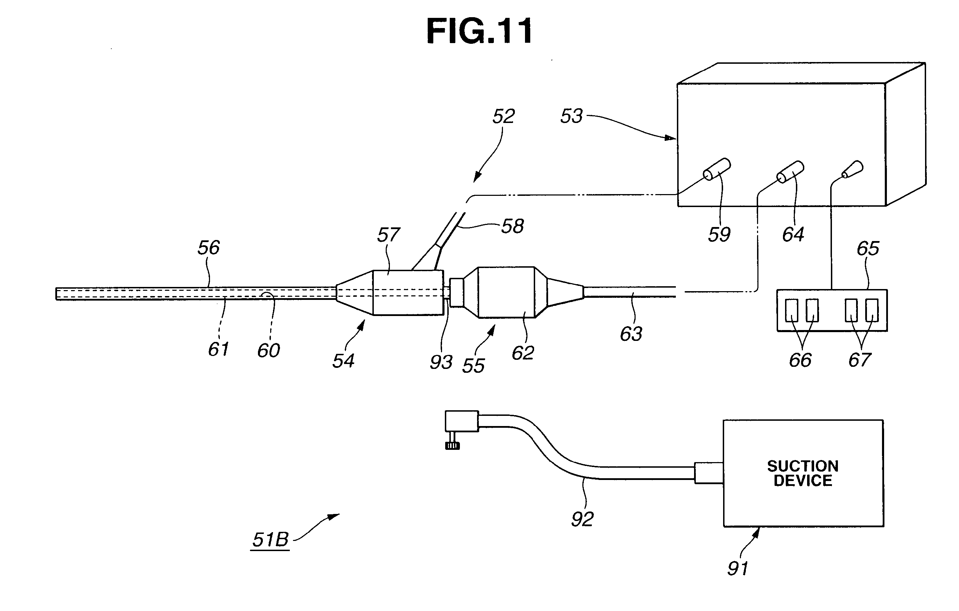

Next, a description is given of a fourth embodiment of the present invention with reference to Fig. 11. It is an object of the fourth embodiment of the present invention to provide a calculus treatment apparatus and a calculus treatment system in which the large calculus is efficiently pulverized and removed.

Fig. 11 shows a calculus treatment system 51B according to the fourth embodiment. As compared with the calculus treatment system 1 shown in Fig. 7, the calculus treatment system 51B further comprises a suction device 91. The front end of a suction tube 92 whose back end is connected to the suction device 91 is detachably connected to a cap portion 93 forming an opening at the back end of the inserting hole 60 in the ultrasonic lithotripsy probe 54.

That is, as shown in Fig. 7 or 11, upon pulverizing the large calculus, the lithotripsy is performed by inserting the probe portion 61 of the mechanical shock lithotripsy probe 55 into (the cap portion 93 forming the opening of) the back end of the inserting hole 60 of the ultrasonic lithotripsy probe 54.

As a result of the above lithotripsy, in the case of the small calculus which does not need to be pulverized by the mechanical shock lithotripsy probe 55, the mechanical shock lithotripsy probe 55 is removed from the inserting hole 60 and the front end of the suction tube 92 is connected to the cap portion 93.

The small-sized calculus is sucked and removed to the outside of the body by setting the suction device 91 to the state for the suction operation. The calculus having the size incapable of suction is pulverized by the ultrasonic probe 54 to reduce the size and the small calculus is sucked and removed to the outside of the body.

In the case of the small calculus, the suction tube 92 is connected to the cap portion 93 without using the mechanical shock lithotripsy probe 55 and the small calculus is pulverized by the ultrasonic probe 54 to have the smaller size. It is sucked and removed to the outside of the body.

When the calculus is pulverized by the ultrasonic probe 54, suction means is set to the suction state, the opening of the distal end of the probe portion 56 in the ultrasonic probe 54 is abutted against the calculus. Thus, the abutting state to the calculus is held, the ultrasonic waves are efficiently transmitted to the calculus, and they are pulverized.

Referring to Fig. 9, in the case of forming the hole 82 to the large calculus 81, the suction means is set to the suction state and the hole 82 is certainly formed.

With the structure and the operation according to the fourth embodiment, since the suction means is provided, the large calculus is efficiently pulverized. Further, the calculus in the body is efficiently removed by suction and removal.

Further, the inserting hole 60 is used for the suction and excretion of the calculus or is used for the insertion of the probe portion 61 in the mechanical shock lithotripsy probe 55. The proper lithotripsy is performed depending on the calculus as the (pulverization) excretion target.

According to a modification of the fourth embodiment, for example, the cap portion 93 may be bifurcated to enable the insertion of the probe portion 61 in the mechanical shock lithotripsy probe 55 on the linear side. Further, the suction tube may be connected to the formation side of another diagonal portion.

In this case, the probe portion 61 in the mechanical shock lithotripsy probe 55 can be inserted and detached while connecting the suction tube.

Further, in this case, the inserting hole 60 may have a notch portion having not only a circular portion of the cross section for inserting the probe portion 61 in the mechanical shock lithotripsy probe 55 in the fitting state, having but also a partly non-fitting portion in which a groove portion for suction in the longitudinal direction is formed. The suction force may be operated to maintain the state for abutting (or stopping) the distal end of the probe portion 61 in the mechanical shock lithotripsy probe 55 to the calculus by suction using the suction means even while the probe portion 61 is inserted.

In the case of stopping to the calculus by using the suction force, the stop operation by the projection 73 is not necessary.

As mentioned above, according to the third and fourth embodiments, the large calculus is efficiently pulverized by detachably inserting the mechanical shock lithotripsy probe 55 into the inserting hole 60 of the ultrasonic lithotripsy probe 54.

That is, the large calculus is efficiently pulverized by setting the stopping state to the calculus by using the projection 73 projected in the side direction from the distal end side of the ultrasonic lithotripsy probe 54.

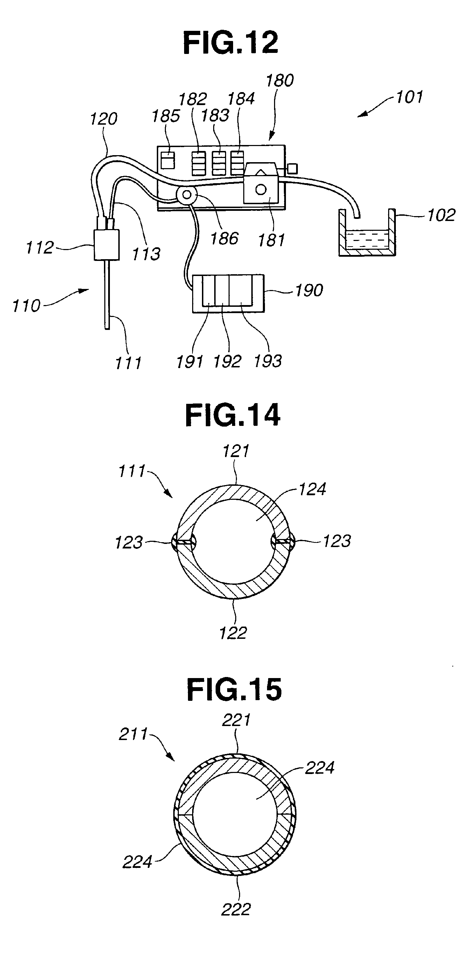

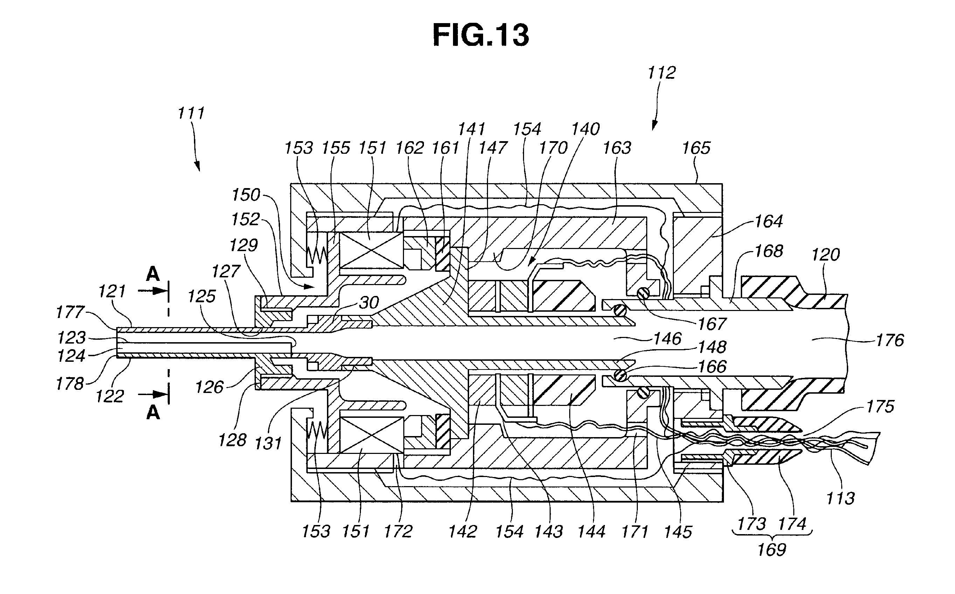

Next, a description is given of a fifth embodiment of the present invention with reference to Figs. 12 to 14.

Referring to Fig. 12, a calculus treatment system 101 comprises a hand piece 110 (as a calculus treatment apparatus), a suction tube 120, a main body 180, and a foot switch 190.

The hand piece 110 comprises a probe 111, an oscillating portion 112, and a cable 113.

The main body 180 comprises a suction pump 181, at least two buttons 182 for setting the ultrasonic output level, at least two buttons 183 for setting the mechanical shock wave output level, at least two buttons 184 for setting the suction output level, a power supply switch 185, and a connector 186.

The foot switch 190 comprises an ultrasonic output pedal 191, an output pedal 192 for outputting the mechanical shock waves, and a suction pedal 193.

A drainage bucket 102 is arranged at one end of the suction tube 120.

Referring to Figs. 13 and 14, the probe 111 comprises an inserting portion 121 for ultrasonic vibration, an inserting portion 122 for mechanical shock wave, sealing and joining members 123 made of an elastic member such as silicone, an ultrasonic vibration transmitting surface 131, a screw tightening portion 129, and a mechanical shock wave transmitting surface 128.

The inserting portion 121 for the ultrasonic vibration and the inserting portion 122 for the mechanical shock wave have the cross sections which are C-shaped, and are joined by the sealing and joining members 123 made of silicone. The probe 111 forms a tube member having an opening portion 124 by combining the inserting portion 121 for the ultrasonic vibration and the inserting portion 122 for the mechanical shock wave.

The proximal end side of the inserting portion 122 for the mechanical shock wave is divided from the inserting portion 121 for the ultrasonic vibration by a surface 125 perpendicular to the tube axis direction of the probe 111.

A flange portion 126 is formed at the outer periphery of the inserting portion 122 for the mechanical shock wave at the proximal end side. A through-hole 127 in which the inserting portion 121 for the ultrasonic vibration is formed to the flange portion 126. The mechanical shock wave transmitting surface 128 is formed to the rear surface on the outer-periphery side of the flange portion 126. The screw tightening portion 129 is formed to the rear side of the mechanical shock waves transmitting surface 128 in the flange portion 126.

A large-diameter portion 130 is formed on the proximal end side of the inserting portion 121 for the ultrasonic vibration. The ultrasonic vibration transmitting surface 131 is formed to the outer periphery on the proximal end side of the large-diameter portion 130.

The oscillating portion 112 comprises an ultrasonic oscillating portion 140, a mechanical shock wave oscillating portion 150, a rubber plate 161 as a member for positioning the ultrasonic oscillating portion 140 and the mechanical shock wave oscillating portion 150, a pin face for fixing 162, casings 163, 164, and 165, O-shaped rings 166 and 167, a cap member 168, and a member 169 for preventing the break-down of the cord.

The ultrasonic oscillating portion 140 comprises a horn 141, a piezoelectric element 142, a pair of electrodes 143, a backing plate 144, and a pair of electric wires 145.

The horn 141 is conically formed, having the ultrasonic vibration transmitting surface 131 of the inserting portion 121 for the ultrasonic vibration at the distal end thereof, a luminal portion 146 which is continuously connected to the opening portion 124 along the central axis, and a flange portion 147 at the outer periphery.

The flange portion 147 of the horn 141 is sandwiched between a step portion 170 of the casing 163 and the rubber plate 161. The pin face for fixing 162 fixes the rubber plate 161 to the casing 163.

The tube portion 148 is extended to the proximal end side of the horn 141. The piezoelectric element 142 and the backing plate 144 are attached to the outer periphery of the tube portion 148. The piezoelectric element 142 is sandwiched between the horn 141 and the backing plate 144. The pair of electrodes 143 is attached to the piezoelectric element 142. One end of the pair of the electric wires 145 is connected to the pair of the electrodes 143. The pair of electric wires 145 is extended to the outside of the casing 163 via the pair of through-holes 171 of the casing 163.

The mechanical shock wave oscillating portion 150 comprises an electromagnet 151, a projecting member 152, a plurality of springs 153 for return to the origin, and a pair of electric wirings 154. The electromagnet 151 is attached to the front side of the pin face for fixing 162 at the inner periphery of the casing 163. The flange portion 155 of the projection member 152 is arranged on the front side of the electromagnet 151 at the inner periphery of the casing 163.

The plurality of springs 153 for return to the origin are inserted between the side surface of the distal end of the flange portion 155 and the inner side surface in the front portion of the casing 163. The pair of electric wirings 154 is extended from the electromagnet 151. The pair of electric wirings 154 is extended to the outside of the casing 163 via the pair of through-holes 172 of the casing 163.

The cap member 168 and the member 169 for preventing the break-down of the cord are attached to the rear side of the casing 165 via the casing 164.

A tube portion 148 of the horn 141 is inserted to the opening on the distal end side of the cap member 168. An O-shaped ring 166 is provided between the cap member 168 and the tube portion 148 of the horn 141.

The distal end side of the cap member 168 is inserted to the opening on the rear side of the casing 163. An O-shaped ring 167 is provided between the cap member 168 and the casing 163.

The member 169 for preventing the break-down of the cord comprises a metal fixing portion 173 and an outside soft portion 174.

The fixing portion 173 is fixed to the casing 164 by screwing.

The pair of electric wires 145 extended to the outside of the casing 163 and the pair of the electric wires 154 are collected as a single cable 113. The cable 113 is extended to the outside via the opening portion 175 of the member 169 for preventing the break-down of the cord.

With the above structure, the ultrasonic oscillating portion 140 is first vibration generating means which can generate the vibration.

The inserting portion 121 for the ultrasonic vibration is a first vibration transmitting member which is long, is connected to the first vibration generating means, and can transmit the vibration generated by the first vibration generating means to the distal end portion thereof.

The mechanical shock wave oscillating portion 150 is a second vibration generating means which can generate the vibration.

The inserting portion 122 for the mechanical shock wave is a second vibration transmitting member which is long, is connected to the second vibration generating means, can transmit to the distal end portion, the vibration generated by the second vibration generating means, and is shaped to form the tube member (probe 111) having a hollow passage by engagement with the first vibration transmitting member.

The sealing and joining members 123 are joining means which is made of an elastic member, joins the first vibration transmitting member to the second vibration transmitting member so that the tube member (probe 111) is formed.

The inserting portion 121 for the ultrasonic vibration is a first vibration transmitting member which has a first treatment portion for treatment to an examinee and can transmit to the first treatment portion, the vibration generated by the first vibration transmitting means.

The inserting portion 122 for the mechanical shock wave is a second vibration transmitting member which has a second treatment portion for treatment to the examinee, can transmit to the second treatment portion, the vibration generated by the second vibration generating means, and can form the tube member (probe 111) having a hollow portion by the engagement with the first vibration transmitting member.

The sealing and joining members 123 seal a joining portion at which the first vibration transmitting member is joined to the second transmitting member with watertightness.

The inserting portion 122 for the mechanical shock wave is a probe for the mechanical shock wave which is formed by longitudinally cutting the pipe in the long-axis direction.

The inserting portion 121 for the ultrasonic vibration is a probe for strong ultrasonic vibration which is formed by longitudinally cutting the pipe in the long-axis direction.

The sealing and joining members 123 are sealing means which forms a hollow portion for suction by adhering both the probes.

Next, a description is given of a method for using the calculus treatment system 101 according to the fifth embodiment. First, the preparation of instruments will be described.

The operator first sticks the ultrasonic vibration transmitting surface 131 of the probe 111 to the distal end of the horn 141 in the oscillating 112 shown in Fig. 13, further sticks the mechanical shock wave transmitting surface 128 to an end surface of the projection member 152, and fixes the probe 111 by the screw using the screw tightening portion 129.

Next, the operator presses one end portion of the suction tube 120 into an end portion of the cap member 168. Further, the other end of the suction tube 120 is arranged in the drainage bucket 102 via the suction pump 181 of the main body 180 shown in Fig. 12. In addition, a plug of the cable 113 is connected to the connector 186.

As a consequence, the preparation for the instruments completes.

Next, a description is given of the calculus treatment using the calculus treatment system 101.

The operator turns on the power supply switch 185 shown in Fig. 12. Thus, power is supplied to the ultrasonic oscillating portion 140 and the mechanical shock wave oscillating portion 150 from the main body 180 via the connector 186 and the cable 113 as shown in Fig. 12 and the pair of electric wires 145 and the pair of electric wires 154 as shown in Fig. 13.

Next, the operator sets the button 182 for setting the ultrasonic output shown in Fig. 12. Hence, circuits in the main body 180 adjusts the amount of power supplied to the electric wire 145 shown in Fig. 13. Further, the operator sets the button 183 for setting the output of the mechanical shock wave shown in Fig. 12. Thus, circuits in the main body 180 adjust the amount of power supplied to the pair of electric wires 154 shown in Fig. 13 and an output time interval thereof. Further, the operator sets the button 184 for setting the suction level shown in Fig. 12. As a result, circuits in the main body 180 adjust the number of revolutions of the motor for driving the suction pump 181. In such a state, the operator presses pedals 191 to 193 of the foot switch 190.

The size and shape of the pedal 191 for the ultrasonic output and the pedal 192 for the output of the mechanical shock wave for the lithotripsy are designed so that the main body 180 is operated only for a pressing period and only one foot results in one-foot pressing or simultaneous two-foot pressing.

On the other hand, once the suction pedal 193 is pressed, the suction starts. Further, it is pressed again and then the suction stops. One-time pressing of the suction pedal 193 enables the circuit in the main body 180 to rotate the suction pump 181 in accordance with an instruction from the button 184 for setting the suction level. Consequently, the partial suction tube 120 attached to the suction pump 181 is drawn for suction.

In this state, the calculus pieces are sucked to the drainage bucket 102 shown in Fig. 12 from the opening portion 124 of the probe 111 shown in Fig. 13 (comprising the inserting portion 121 for the ultrasonic vibration, the inserting portion 122 for the mechanical shock wave, and the sealing and joining members 123 made of silicone for regulating the two inserting portions 121 and 122 in the watertight state so that they are freely moved at some degree) via the luminal portion 146 of the ultrasonic oscillating portion 140 positioned coaxially to the opening portion 124, the luminal portion 176 of the cap member 168, and the suction tube 120 pressed in the cap member 168.

Next, the pressing of the pedal 191 for the ultrasonic output enables the circuit in the main body 180 to supply proper power to the piezoelectric element 142 of the ultrasonic oscillating portion 140 via the connector 186 and the cable 113 and the pair of electric wires 145 and the pair of electric wires 143 shown in Fig. 13 in accordance with the instruction from the button 182 for setting the ultrasonic output level. As a result of the piezoelectric effect, the ultrasonic oscillating portion 140 starts the vibration.

The vibration energy is transmitted to the inserting portion 121 for the ultrasonic vibration of the probe 111 via the ultrasonic vibration transmitting surface 131 and, thus, the distal end 177 of the probe 111 pulverizes the calculus due to cavitation phenomenon.

Next, the pressing of the pedal 192 for the output of the mechanical shock waves enables the circuit in the main body 180 to supply proper power to the electromagnet 151 of the mechanical shock wave oscillating portion 150 via the connector 186 and the cable 113 and the pair of electric wires 154 shown in Fig. 13 at a proper time interval in accordance with the instruction from the button 183 for setting the mechanical shock wave output. As a result, the mechanical shock wave oscillating portion 150 is operated.

Specifically, the mechanical shock wave oscillating portion 150 first enters an on-power state, and the electromagnet 151 projects, with great force, the inserting portion 122 for the mechanical shock wave of the probe 111 to the distal end 178 side of the probe 111 via the metal projection member 152 and the mechanical shock wave transmitting surface 128.

Secondly, the mechanical shock wave oscillating portion 150 enters an off-power state, and the plurality of springs 153 for return to the origin are projected. Then, the projection member 152 and the inserting portion 122 for the mechanical shock wave are returned to the origin. By repeating the first and second operations, the mechanical shock waves are transmitted to the distal end 178 of the probe 111 for lithotripsy.

The operation other than the foregoing will complementarily be described. The rubber 161 and the pin face for fixing 162 fix the ultrasonic oscillating portion 140 in the casings 163, 164, and 165. The casings 163 to 165 and the O-shaped rings 166 and 167 prevent the flow of liquid and dirt to the inner components from the outside and isolate from the outside, the inner components in which the current flows.

As mentioned above, the lithotripsy using the mechanical vibration (shock) and the lithotripsy using the ultrasonic vibration are easily switched and are effectively used according to the first embodiment. The calculus in the coelom is pulverized, sucked, and removed. Accordingly, the pulverization energy is appropriately used for the treatment depending on the treatment target.

Further, the probe 111 is relatively simply made thinner in diameter. This structure does not make a sacrifice of the suction function as compared with the conventional pipe-shaped probe.

Conventionally, the higher medical benefit needs a plurality of types of lithotripsy apparatuses. However, the present invention may use only the calculus treatment system 101 and does not need the exchanging work during the operation. Thus, the operability is improved and the working time is reduced.

Fig. 15 is a cross-sectional view showing the hand piece portion according to the sixth embodiment of the present invention.

The sixth embodiment shown in Fig. 15 is obtained by modifying only the cross-sectional shape of the probe (cross section by an A-A line shown in Fig. 13). Other system structures (main body 180, the oscillating portion 112, the suction tube 120, and the foot switch 190) are the same as those according to the fifth embodiment and therefore they are not described. The seventh to ninth embodiments of the present invention, which will be described later, are similar to the sixth embodiment.

Referring to Fig. 15, a probe 221 comprises an inserting portion 221 for the ultrasonic vibration, an inserting portion 222 for the mechanical shock wave, and a coating tube 223 made of silicone.

The inserting portion 221 for the ultrasonic vibration and the inserting portion 222 for the mechanical shock wave have C-shaped cross sections, and are coated with a coating tube 223 made of silicone. The probe 211 forms a suction tube 224 by attaching the inserting portion 221 for the ultrasonic vibration and the inserting portion 222 for the mechanical shock wave.

Next, the operation according to the sixth embodiment will be described.

According to the sixth embodiment, the inserting portion 221 for the ultrasonic vibration transmits the ultrasonic vibrations, the inserting portion 222 for the mechanical shock wave transmits the mechanical shock waves, and the calculus is sucked by the suction tube 224 formed by attaching the C-shaped cross sections of the inserting portion 221 for the ultrasonic vibration and the inserting portion 222 for the mechanical shock wave. The coating tube 223 maintains the attaching state of the inserting portion 221 for the ultrasonic vibration and the inserting portion 222 for the mechanical shock wave, and prevents the leakage from the suction tube 224.

The sixth embodiment has the same advantages as those according to the fifth embodiment.

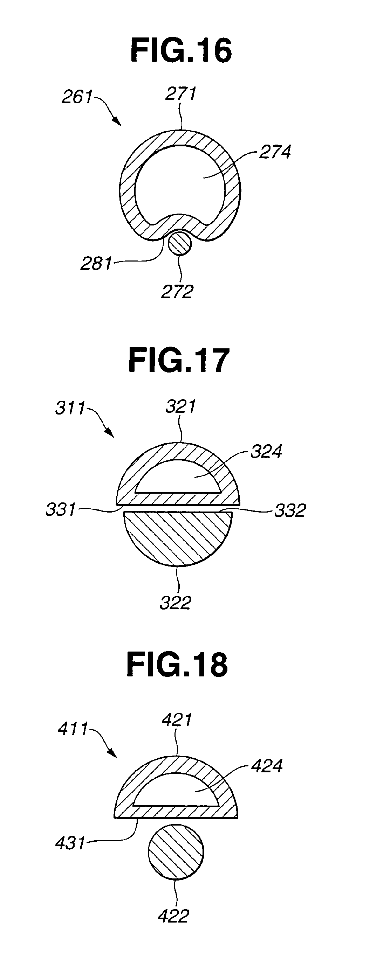

Fig. 16 is a cross-sectional view showing a hand piece portion according to the seventh embodiment of the present invention.

Referring to Fig. 16, a probe 261 comprises an inserting portion 271 for the ultrasonic vibration and an inserting portion 272 for mechanical shock wave.

The inserting portion 271 for the ultrasonic vibration forms a suction tube 274 as a single member. The cross-section of the inserting portion 271 for the ultrasonic vibration is shaped by bending a part of the ring and by forming a hollow portion 281. The inserting portion 272 for the mechanical shock wave is best fit to the hollow portion 281 of the inserting portion 271 for the ultrasonic vibration. The cross section of the inserting portion 272 for the mechanical shock wave is circular-shaped and has no space therein.

With the above structure, the inserting portion 271 for the ultrasonic vibration is a probe for strong ultrasonic vibration having a hollow portion for suction having different cross sections.

The inserting portion 272 for the mechanical shock wave is a probe for the mechanical shock wave which has no space therein to contact with at least a part of the outer periphery of the probe for the strong ultrasonic vibration.

Next, the operation according to the seventh embodiment will be described.

According to the seventh embodiment, the inserting portion 271 for the ultrasonic vibration transmits the ultrasonic vibrations, the inserting portion 272 transmits the mechanical shock waves, and the suction is performed by the suction tube 274 of the inserting portion 271 for the ultrasonic vibration.

According to the seventh embodiment, the almost same advantages as those according to the fifth embodiment are obtained.

Fig. 17 is a cross-sectional view showing a hand piece portion according to the eighth embodiment of the present invention.

Referring to Fig. 17, a probe 311 comprises an inserting portion 321 for the ultrasonic vibration and an inserting portion 322 for the mechanical shock wave.

The inserting portion 321 for the ultrasonic vibration forms a suction tube 324 as a single member. The inserting portion 321 for the ultrasonic vibration has a semicircular-shaped cross section with the suction tube 324 formed therein. The inserting portion 322 for the mechanical shock wave has a semicircular-shaped cross section without space therein. The inserting portion 322 for the mechanical shock wave has a diameter slightly smaller than that of the inserting portion 321 for the ultrasonic vibration. In the inserting portion 321 for the ultrasonic vibration and the inserting portion 322 for the mechanical shock wave, plane portions 331 and 332 are faced each other. As a result, the entire cross section of the probe 311 is circular.

Next, the operation according to the eighth embodiment will be described.

According to the eighth embodiment, the inserting portion 321 for the ultrasonic vibration transmits the ultrasonic vibrations, the inserting portion 322 transmits the mechanical shock waves, and the suction is performed by the suction tube 324 of the inserting portion 321 for the ultrasonic vibration.

According to the eighth embodiment, the almost same advantages as those according to the fifth embodiment are obtained.

Fig. 18 is a cross-sectional view showing a hand piece portion according to the ninth embodiment of the present invention.

Referring to Fig. 18, a probe 411 comprises an inserting portion 421 for the ultrasonic vibration and an inserting portion 422 for the mechanical shock wave.

The inserting portion 421 for the ultrasonic vibration forms a suction tube 424 as a single member. The inserting portion 421 for the ultrasonic vibration has the cross section which is semicircular-shaped and has the suction tube 424. The inserting portion 422 for the mechanical shock wave has a circular-shaped cross section without space therein. The inserting portion 422 for the mechanical shock wave is arranged near the plane portion 431 of the inserting portion 421 for the ultrasonic vibration.

Next, the operation according to the ninth embodiment will be described.

According to the ninth embodiment, the inserting portion 421 for the ultrasonic vibration transmits the ultrasonic vibrations, the inserting portion 422 for the mechanical shock wave transmits the mechanical shock waves, and the suction is performed in the suction tube 424 of the inserting portion 421 for the ultrasonic vibration.

According to the ninth embodiment, the almost same advantages as those according to the fifth embodiment are obtained.

The materials of the sealing and joining member 123 and the coating tube 224 are not limited to silicone and may be made of another member such as natural rubber, which can absorb vibration and seal with watertightness according to the fifth and sixth embodiments with reference to Figs. 12 to 15.

The two C-shaped members (inserting portion for the ultrasonic vibration and the inserting portion for the mechanical shock wave) are jointed to form the cylindrical probe according to the fifth and sixth embodiments with reference to Figs. 12 to 15. However, the cross section of the probe is not limited to be circular and, advantageously, it may be triangular, quadrangular, or another-shaped.

Further, the probe comprises the two vibration transmitting members (inserting portion for the ultrasonic vibration and the inserting portion for the mechanical shock wave) according to the fifth to ninth embodiments with reference to Figs. 12 to 18. However, the probe may comprise three or more vibration transmitting members.

Having described the preferred embodiments of the invention referring to the accompanying drawings, it should be understood that the present invention is not limited to those precise embodiments and various changes and modifications thereof could be made by one skilled in the art without departing from the spirit or scope of the invention as defined in the appended claims.

## Claims

1. A calculus treatment apparatus **characterized by** comprising:
a first probe which transmits first mechanical energy to a distal end side thereof and pulverizes a calculus by the first mechanical energy;
a first mechanical energy generating means which is arranged on a proximal end side of the first probe and generates the first mechanical energy;
a second probe which transmits to a distal end side thereof, second mechanical energy different from the first mechanical energy and pulverizes the calculus by the second mechanical energy; and
a second mechanical energy generating means which is arranged on a proximal end side of the second probe and generates the second mechanical energy different from the first mechanical energy,
**characterized in that** a probe arrangement structure is provided in which the first probe and the second probe are arranged substantially coaxially or concentrically.

2. A calculus treatment apparatus according to Claim 1, **characterized in that** in the probe arrangement structure, the second probe is inserted in a hollow portion formed in the first probe.

3. A calculus treatment apparatus according to Claim 1, **characterized in that** the arrangement structure is formed by dividing a cylindrical-shaped or circular-tube-shaped structure in the longitudinal direction so that the first probe and the second probe have substantially the same central axis.

4. A calculus treatment apparatus according to Claim 1, **characterized in that** in the probe arrangement structure, the second probe is detachably inserted in a hollow portion formed in the first probe.

5. A calculus treatment apparatus according to Claim 1, **characterized in that** the first mechanical energy generating means generates the mechanical energy by magnetic force.

6. A calculus treatment apparatus according to Claim 1, **characterized in that** the first mechanical energy generating means generates the mechanical energy by ultrasonic vibration.

7. A calculus treatment apparatus according to Claim 1, **characterized in that** the first mechanical energy generating means generates the mechanical energy by magnetic force and the second mechanical energy generating means generates the mechanical energy by ultrasonic vibration.

8. A calculus treatment apparatus according to Claim 1, **characterized in that** a distal end of the second probe is positioned within or in a part of a moving range of a distal end of the first probe by the first mechanical energy.

9. A calculus treatment apparatus according to Claim 1, **characterized in that** the probe arrangement structure has a hollow passage for inserting a pulverized calculus.

10. A calculus treatment apparatus according to Claim 2, **characterized in that** the second probe has a hollow passage for inserting a pulverized calculus.

11. A calculus treatment apparatus according to Claim 7, **characterized in that** the distal ends of the first and second probes are arranged so that the entire or at least a part of a stroke width of the ultrasonic vibration of the distal end of the second probe is overlapped to a moving stroke width upon pulverization using the distal end of the first probe.

12. A calculus treatment apparatus according to Claim 1, **characterized in that** the first probe is a lithotripsy probe which is driven by magnetic force.

13. A calculus treatment apparatus according to Claim 1, **characterized in that** the first probe is an ultrasonic probe which is driven by ultrasonic waves.

14. A calculus treatment apparatus according to Claim 3, **characterized in that** the first probe is jointed to the second probe, thus forming a cylindrical member for inserting the pulverized calculus.

15. A calculus treatment apparatus according to Claim 2, **characterized in that** the first probe is an ultrasonic probe which is driven by ultrasonic waves.

16. A calculus treatment apparatus according to Claim 2, **characterized in that** the first probe is a lithotripsy probe which is driven by magnetic force.

17. A calculus treatment apparatus according to Claim 4, **characterized in that** a suction device can be connected to a proximal end of the hollow portion formed in the first probe.

18. A calculus treatment apparatus according to Claim 1, **characterized in that** the first mechanical energy generating means and the second mechanical energy generating means are arranged adjacently in the longitudinal direction of the first probe and second probe.

19. A calculus treatment apparatus according to Claim 1, **characterized in that** the first mechanical energy generating means has a hollow portion for inserting the second probe.

20. A calculus treatment apparatus according to Claim 4, **characterized in that** a projection portion projected in a side direction of the first probe is arranged at the distal end of the first probe.

21. A calculus treatment system **characterized by** comprising:
a first probe which transmits first mechanical energy to a distal end side thereof and pulverizes a calculus by the first mechanical energy;
a first mechanical energy generating means which is arranged on a proximal end side of the first probe and generates the first mechanical energy;
a second probe which transmits to a distal end side thereof, second mechanical energy different from the first mechanical energy and pulverizes the calculus by the second mechanical energy;
a second mechanical energy generating means which is arranged on a proximal end side of the second probe and generates the second mechanical energy different from the first mechanical energy; and
a driving device which supplies electric driving energy to generate the first and second mechanical energy in the first and second mechanical energy generating means,
**characterized in that** a probe arrangement structure is provided in which the first probe and the second probe are arranged substantially coaxially or concentrically.

22. A calculus treatment system according to Claim 21, **characterized in that** in the probe arrangement structure, the second probe is inserted in a hollow portion formed in the first probe.

23. A calculus treatment system according to Claim 21, **characterized in that** the arrangement structure is formed by dividing a cylindrical-shaped or circular-tube-shaped structure in the longitudinal direction so that the first probe and the second probe have substantially the same central axis.

24. A calculus treatment system according to Claim 21, **characterized in that** in the probe arrangement structure, the second probe is detachably inserted in a hollow portion formed in the first probe.

25. A calculus treatment system according to Claim 21, **characterized in that** the first mechanical energy generating means generates the mechanical energy by magnetic force.

26. A calculus treatment system according to Claim 21, **characterized in that** the first mechanical energy generating means generates the mechanical energy by ultrasonic vibration.

27. A calculus treatment system according to Claim 21, **characterized in that** the probe arrangement structure has a hollow passage for inserting a pulverized calculus and a suction device can be connected to the hollow passage.
